# EUROPEAN PATENT APPLICATION

(11) **EP 4 517 303 A1**
(43) Date of publication of application: **05.03.2025**
(21) Application number: 23796414.3
(22) Date of filing: 26.04.2023
(51) Int. Cl.: G01N 21/61, G01N 21/359

(54) **OPTICAL GAS SENSOR DEVICE**

(30) Priority: 27.04.2022 JP 2022073483
(71) Applicant: MITSUMI ELECTRIC CO., LTD., Tama-Shi, Tokyo 206-8567 (JP)
(72) Inventor: OKAMOTO Mitsuhiro, Tama-shi, Tokyo 206-8567 (JP); EHARA Masato, Tama-shi, Tokyo 206-8567 (JP); NAKAMURA Akira, Tama-shi, Tokyo 206-8567 (JP); FURUKAWA Kenichi, Tama-shi, Tokyo 206-8567 (JP)
(74) Representative: Epping - Hermann - Fischer
(86) International application number: PCT/JP2023/016399
(87) International publication number: WO 2023/210674

(57) **Abstract**

The present invention improves the accuracy of detection of a gas being detected, by freely forming an optical path in three dimensions. An optical gas sensor device 100 comprises: a light source 2 that emits infrared radiation to a gas being detected; an optical filter 3 that transmits infrared radiation having a wavelength corresponding to the absorption wavelength of the gas being detected; a light receiving unit 4 that detects infrared radiation incoming through the optical filter 3 and generates a detection signal; and a cover 1 that covers the light source **2,** the optical filter 3, and the light receiving unit 4. The cover 1 has a light guiding part 13 that guides the infrared radiation incoming from the light source 2 by reflections on the inner surface thereof to the light receiving unit 4 through the optical filter 3, has a pipe shape, and has a circular or oval cross-section perpendicular to the axial direction thereof.

## Description

### Technical Field

The present invention relates to an optical gas sensor device.

### Background Art

Conventionally, gas sensors using a non-dispersive infrared (NDIR) absorption method are known. NDIR gas sensors utilize the property that many gases each absorb a specific infrared wavelength. The NDIR gas sensor emits infrared rays to a detection target gas, detects which wavelength is absorbed and how much, and measures the concentration of the detection target gas. For example, the gas sensor includes an infrared light emitter and an infrared light receiver and detects the concentration of the detection target gas on an optical path of the light emitter and the light receiver.

The light receiver detects infrared rays absorbed by the detection target gas, and the gas sensor calculates the gas concentration based on the difference between the detection before and after the absorption. Therefore, theoretically, the longer the optical path is, the more distinct the difference is by the light absorption amount, and detection accuracy (measurement accuracy) of the gas concentration is increased.

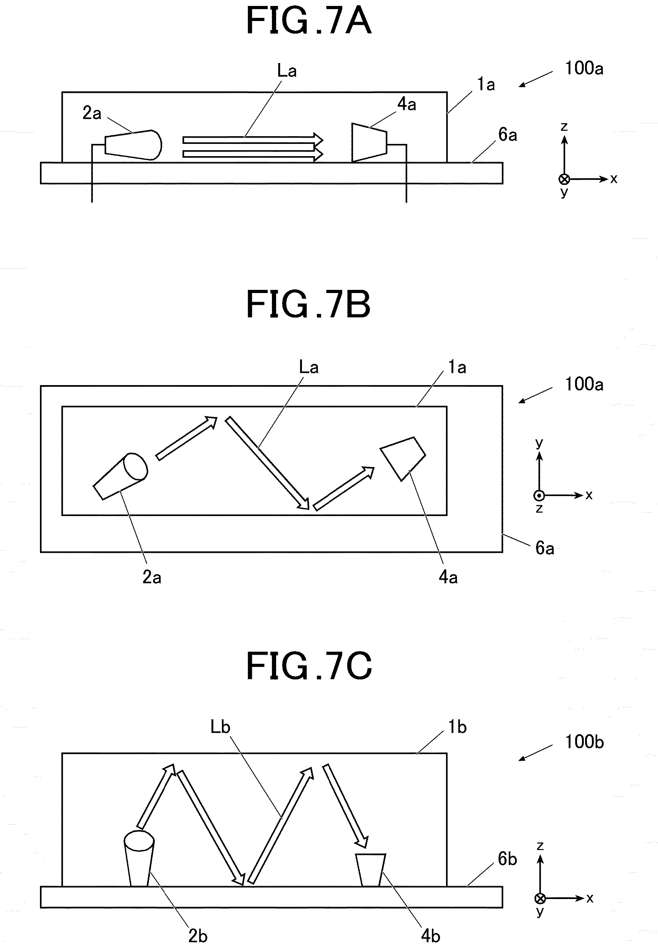

Here, a conventional NDIR optical gas sensor device with a longer optical path is described with reference to FIG.7A to FIG.7C. FIG.7A is a lateral schematic view of a conventional optical gas sensor device 100a. FIG.7B is a top schematic view of the conventional optical gas sensor device 100a. FIG.7C is a lateral schematic view of a conventional optical gas sensor device 100b. In FIG.7A to FIG.7C, three dimensional axes of x, y, and z are shown.

As shown in FIG.7A and FIG.7B, the conventional optical gas sensor device 100a includes, on a board 6a placed on the x-y plane, a light source 2a that emits infrared rays, a light receiver 4a that is arranged such that the optical axis is substantially opposite the light source 2a on the x-y plane when viewed from the lateral surface (the x-z plane) and that detects infrared rays, and a cover 1a that covers the light source 2a, the light receiver 4a, and the detection target gas. The light source 2a and the light receiver 4a are dual inline package (DIP) components. A DIP component has an external shape with multiple metal connection terminals sticking out of two sides of a plastic or ceramic body and extending downward.

The optical path La, which is emitted by the light receiver 4a, reflected on the inner surface of the cover 1a, and received by the light receiver 4a, is reflected in the x, y directions but not in the z direction. Therefore, the planar size of the board 6a is increased, and the board size is restricted.

As shown in FIG.7C, the conventional optical gas sensor device 100b includes, on a board 6b placed on the x-y plane, a light source 2b that emits infrared rays, a light receiver 4b that is arranged such that the optical axis is substantially parallel to the light source 2b when viewed from the lateral surface (the x-z plane) and that detects infrared rays, and a cover 1b that covers the light source 2b, the light receiver 4b, and the detection target gas. The light source 2b and the light receiver 4b are DIP components.

The optical path Lb of infrared rays, which are emitted by the light receiver 4a, reflected on the inner surface of the cover 1a and the board 6b, and received by the light receiver 4a, is reflected substantially in the z direction. Therefore, the inside of the cover 1b needs to have a distance in the z direction.

According to the optical gas sensor devices 100a, 100b, when the distance between the light source and the light receiver is short or when a small board is required due to area restrictions, the distance is increased by increasing the number of reflections of the optical path. However, to form a cover that efficiently reflects light, calculations of the angle and curvature of the reflection surface are required. Designing such a cover requires skills.

Further, for a configuration in which the optical gas sensor devices 100a and 100b are combined (the light source and the light receiver are three-dimensionally angled with respect to the board and mounted), the angle of the light source and light receiver needs to be controlled. It is therefore difficult to mount the light source and the light receiver. Practically, it is conceivable that the light source and the light receiver as DIP components are mounted on a cover having an angled inside and that the light source, the light receiver, and the cover are together mounted on the board. However, such a structure requires additional soldering if the board has surface-mounted components, and it is difficult to reduce costs.

To deal with this, there is known an optical absorption gas sensor that includes a light emitting diode; an annular radiation guide that includes curved portions that can be curved around an axis of one side of the rectangular cross section and that guides near infrared rays emitted by the light emitting diode; and a mid infra-red emitting photodiode that detects mid infrared rays emitted by the annular radiation guide (see Patent document 1).

### CITATION LIST

### Patent Literature

Patent document 1: Japanese Unexamined Patent Application Publication No. 2013-517467

### SUMMARY OF INVENTION

### Technical Problem

However, according to the optical absorption gas sensor described in Patent document 1, the curving direction (angle) is two-dimensional, and it is difficult to reduce the height of the gas sensor. There has been a demand for freely forming optical paths in three dimensions. Further, since the optical absorption gas sensor described in Patent document 1 has a rectangular cross section perpendicular to the axial direction of the annular radiation guide, reflection angles of optical paths of infrared rays are limited. Therefore, misalignment of the light source and the light receiver may cause a light quantity loss of infrared rays and may cause low detection accuracy of the detection target gas.

An object of the present invention is to freely form optical paths in three dimensions and increase detection accuracy of the detection target gas.

### Solution to Problem

To solve the above problem, according to the present invention, an optical gas sensor device includes:
a light source that emits infrared rays to a detection target gas;
an optical filter that transmits infrared rays having a wavelength corresponding to an absorption wavelength of the detection target gas;
a light receiver that detects infrared rays entering via the optical filter and generates a detection signal; and
a cover that covers the light source, the optical filter, and the light receiver, wherein
the cover includes a light guide part that guides infrared rays entering from the light source to the light receiver via the optical filter by reflecting the infrared rays on an inner surface;
the light guide part has a pipe shape; and
a cross section of the light guide part perpendicular to an axial direction is circular or oval.

### Advantageous Effects of Invention

According to the present invention, optical paths can be freely formed in three dimensions, and detection accuracy of the detection target gas can be increased.

### BRIEF DESCRIPTION OF DRAWINGS

[FIG.1] This is a schematic diagram of an optical gas sensor device according to an embodiment of the present invention.
[FIG.2] This is an external perspective view of the optical gas sensor device.
[FIG.3] This is a cross-sectional view of the optical gas sensor device along the III-III line in FIG.2.
[FIG.4] This is a cross-sectional view of the optical gas sensor device along the IV-IV line in FIG.2.
[FIG.5] This is a partially transparent perspective view of the optical gas sensor device.
[FIG.6] This is a perspective view of a light source.
[FIG.7A] This is a schematic view of the lateral side of a known optical gas sensor device.
[FIG.7B] This is a schematic view of the top side of the known optical gas sensor device.
[FIG.7C] This is a schematic view of the lateral side of another known optical gas sensor device.

### DESCRIPTION OF EMBODIMENTS

Hereinafter, an embodiment of the present invention is described in detail with reference to the accompanying figures. However, the scope of the invention is not limited to the illustrated examples.

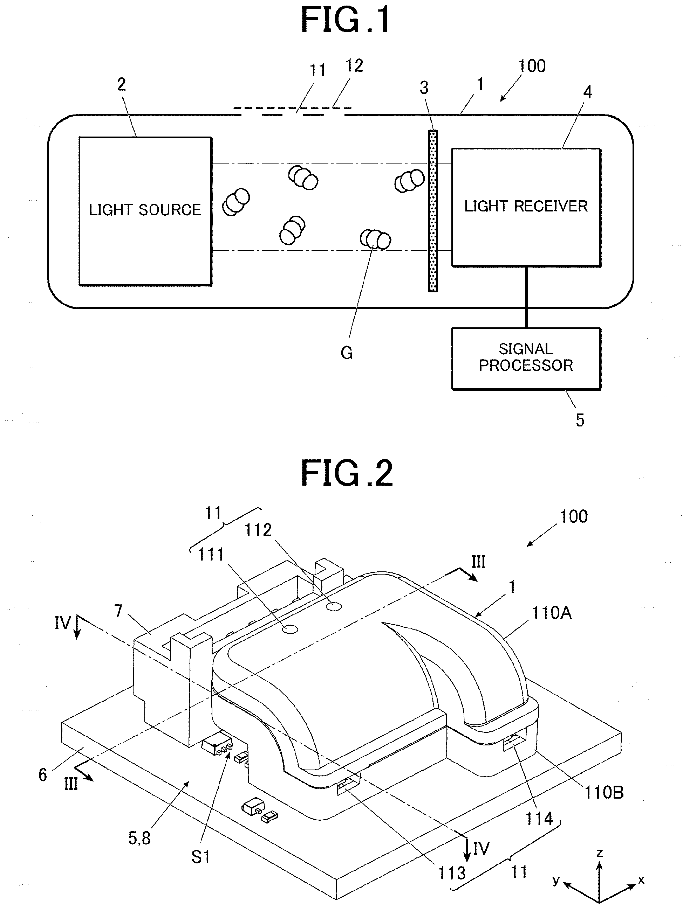

The embodiment according to the present invention is described with reference to FIG.1 to FIG.6. First, a schematic configuration of an optical gas sensor device 100 according to the present embodiment is described with reference to FIG.1. FIG.1 is a schematic diagram of the optical gas sensor device 100 in this embodiment.

As shown in FIG.1, the optical gas sensor device 100 in this embodiment includes a cover 1, a light source 2, an optical filter 3, a light receiver 4, and a signal processor 5. The optical gas sensor device 100 is an NDIR gas sensor. The light source 2 emits infrared rays (emits light) and irradiates, with the infrared rays, a detection target (measurement target) gas G in the cover 1 through an optical path in the cover 1. The molecules of the detection target gas G in the optical path absorb the infrared rays, so that a less amount of light reaches the light receiver 4. The light receiver 4 detects, via the optical filter 3, the infrared rays partially absorbed by the detection target gas G. The signal processor 5 processes the detection signal, detects (measures) the concentration of the detection target gas G, and outputs the concentration. The cover 1 has gas introduction ports 11 as gas introduction parts that are inlets/outlets of the detection target gas G. To prevent entrance of foreign matters from outside, a gas filter 12 (e.g., a metal mesh filter or a porous resin film) is put on the gas introduction ports 11.

Specifically, according to the optical gas sensor device 100, the optical filter 3 filters infrared rays emitted by the light source 2 to the detection target gas G, and the light receiver 4 receives the filtered infrared rays. The optical filter 3 is positioned near the light receiver 4 in the upstream of the light receiver 4 on the optical path. Since the filtering is performed on the light receiving surface of the light receiver 4 in this configuration, the area of the optical filter 3 can be reduced and the cost is reduced, as compared with a configuration in which infrared rays emitted by the light source 2 are filtered by the optical filter 3 and then delivered toward the detection target gas G. Further, since the light receiver 4 does not receive unfiltered infrared rays emitted by sources other than the light source 2, the signal-to-noise ratio (SN ratio) as a sensor is increased. However, the optical gas sensor device 100 may be configured to filter infrared rays emitted by the light source 2 with the optical filter 3 and deliver the filtered infrared rays toward the detection target gas G. In this embodiment, the optical path is designed such that infrared rays are reflected on the inner surface of the cover 1 before arriving at the light receiver 4 and being received by the light receiver 4 via the optical filter 3, instead of arriving at the light receiver 4 directly from the light source 2. It is preferable that the inner surface of the cover 1 have a high reflectance to increase light (infrared rays) use efficiency.

The optical gas sensor device 100 can be used for detecting carbon monoxide, propane, methane, butane, ammonia, oxygen disulfide, nitrogen dioxide, nitric oxide, ozone, sulfur hexafluoride, difluoromethane, hydrochlorofluorocarbons (HCFC), hydrofluorocarbons (HFC), perfluorocarbons (PFCs), ethylene, or the like, as the detection target gas G.

In such a case, it is preferable that the optical gas sensor device 100 detect absorption of infrared rays having the most absorbed wavelength among the absorption wavelengths of the detection target gas. For example, in a case of detecting carbon dioxide (CO₂) as the concentration detection target gas G, the optical gas sensor device 100 detects the absorption of infrared rays having the wavelength of 4.26 [µm], which is the most absorbed wavelength among the absorption wavelengths of CO₂.

The optical gas sensor device 100 outputs the concentration of the detected gas G or the value corresponding to the concentration and various state signals based on the concentration of the detected gas G to an apparatus that performs processing based on the concentration of the detected gas G or the value corresponding to the concentration and the state of the optical gas sensor device 100 (e.g., malfunction). The various status signals are, for example, a malfunction signal indicating a malfunction state of the optical gas sensor device 100, a warning signal indicating an abnormal state (warning state) in which the concentration of the detected gas G needs to be warned, and a monitoring signal (normal signal) indicating that the concentration of the detected gas G is in a normal state. When the apparatus is an alarm apparatus, the apparatus outputs various alarms corresponding to the various signals received from the optical gas sensor device 100. The various alarms are, for example, an alarm indicating a malfunction of the optical gas sensor device 100 based on the malfunction signal, and an alarm indicating an abnormal concentration of the detected gas G based on the warning signal. The optical gas sensor device 100 may be included in the apparatus.

As the apparatus, following apparatuses can be applied: a household air conditioning apparatus, a household water heater, an industrial air conditioning apparatus, an automotive air conditioning apparatus, a freezer, a refrigerator, a refrigerator showcase, an air cleaner, a household combustible gas leak alarm apparatus, a household toxic gas alarm apparatus, a household environmental monitoring apparatus, an industrial combustible gas leak alarm apparatus, an industrial toxic gas alarm apparatus, an industrial gas process monitoring apparatus, a CO₂ concentration measuring apparatus for horticultural facilities, a CO₂ measuring apparatus for plant factories, a CO₂ sealing apparatus for food packaging, and an ethylene gas concentration measuring apparatus for food warehouses. For example, an ammonia monitoring apparatus and a gas leakage alarming apparatus can be applied. The ammonia monitoring apparatus is for an ammonia tank that stores ammonia as fuel or as a carrier for hydrogen, which is the fuel of decarbonization.

In particular, for an apparatus that directly manages the concentration of the detected gas G, such as a household environmental monitoring apparatus, a CO₂ concentration measuring apparatus for horticultural facilities, a CO₂ measuring apparatus for plant factories, and an ethylene gas concentration measuring apparatus for food warehouses, the optical gas sensor device 100 outputs signals corresponding to the concentration of the detected gas G or the value corresponding to the concentration to the apparatus.

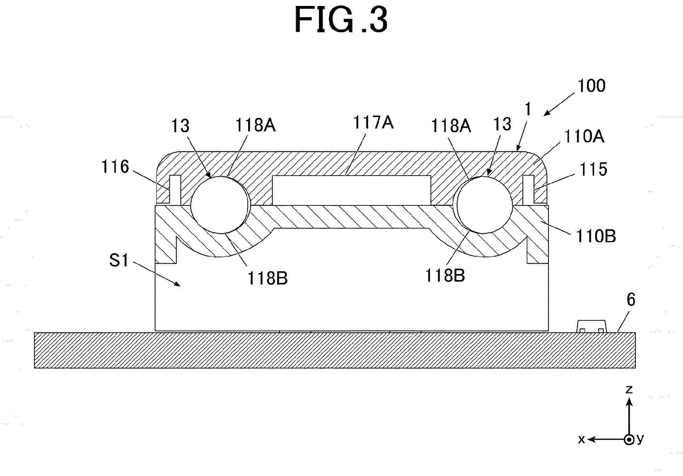

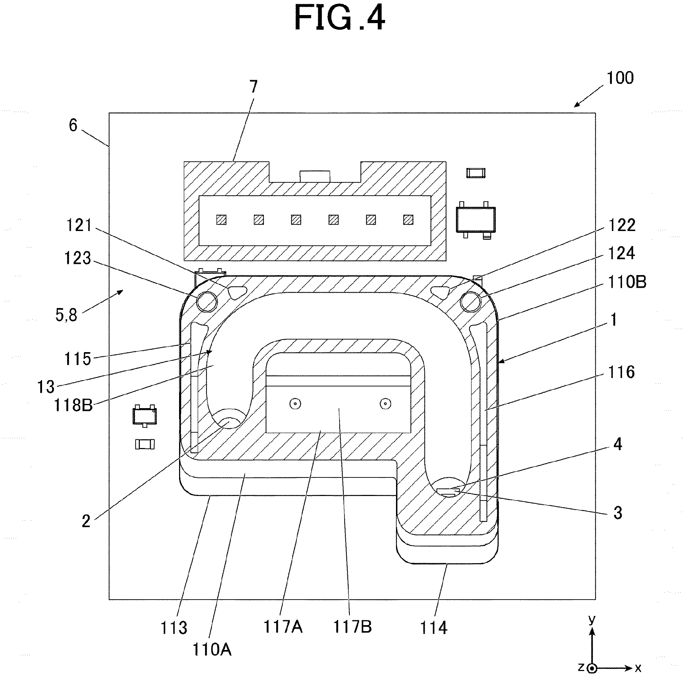

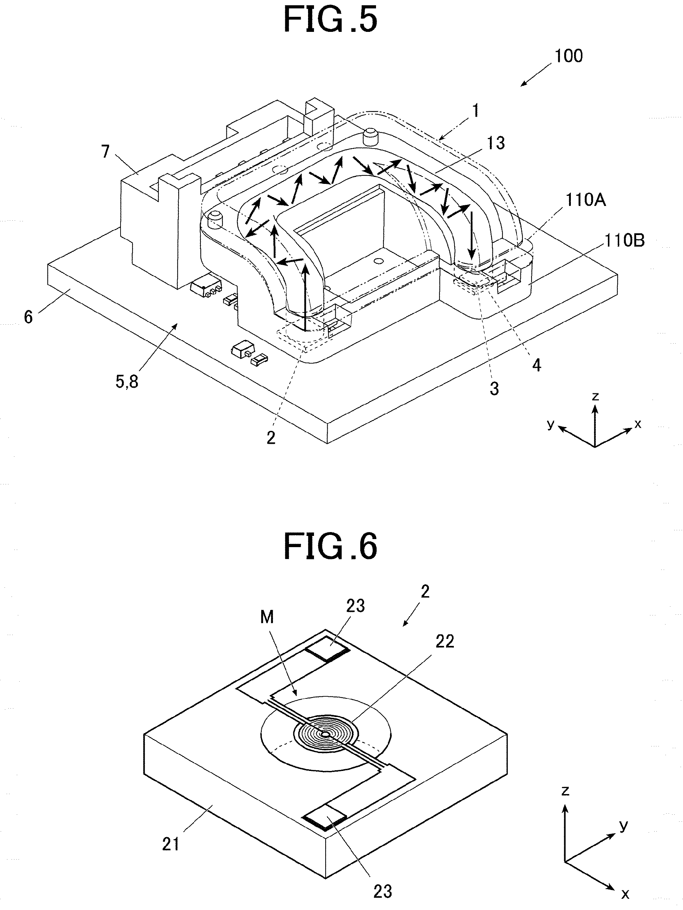

Next, a detailed configuration of the optical gas sensor device 100 is described with reference to FIG.2 to FIG.6. FIG.2 is an external perspective view of the optical gas sensor device 100. FIG.3 is a cross-sectional view of the optical gas sensor device 100 along the III-III line in FIG.2. FIG.4 is a cross-sectional view of the optical gas sensor device 100 along the IV-IV line in FIG.2. FIG.5 is a partially transparent perspective view of the optical gas sensor device 100. FIG.6 is a perspective view of the light source 2.

As shown in FIG.2, the optical gas sensor device 100 includes the cover 1, the light source 2, the optical filter 3, the light receiver 4, the signal processor 5, a board 6, a connector 7, and circuit elements 8, for example.

FIG.2 shows an x axis, a y axis, and a z axis. These three axes are also shown in FIG.3 to FIG.5 in the same manner. In

FIG.2, FIG.4 and FIG.5, illustration of the gas filter 12 is omitted.

FIG.3 shows the cross-sectional view of the optical gas sensor device 100 along the III-III line in FIG.2, wherein the cross section is along the x-z plane. FIG.4 shows the cross-sectional view of the optical gas sensor device 100 along the IV-IV line in FIG.2, wherein the cross section is along the x-y plane. FIG.5 shows the perspective view of the optical gas sensor device 100, wherein the cover 1 of the optical gas sensor device 100 is transparent.

The cover 1 is mounted on the +Z side surface of the board 6 to cover (house) the light source 2, the optical filter 3, and the light receiver 4. The cover 1 forms a hollow part that can accommodate the detection target gas G. The detection target gas G is let into and let out from the space through the gas introduction ports 11. The base body of the cover 1 is made of resin, for example.

As shown in FIG.2, the cover 1 has cover parts 110A, 110B. The cover part 110A is an upper side (+z direction side) part and is fitted to the cover part 110B. The cover part 110B is a lower side (-z direction side) part and is fitted to the cover part 110A.

As shown in FIG.3, the cover 1 has a light guide part 13 as a hollow part into which the detection target gas G is introduced. The light guide part 13 has a pipe shape. The cross section of the light guide part 13 perpendicular to the axial direction is circular. The cover part 110A has a half-pipe section 118A. The cover part 110B has a half-pipe section 118B. By the combination of the half-pipe sections 118A and 118B, the light guide part 13 is formed. Thus, the cover 1 has the cover parts 110A, 110B into which the cover 1 is divided along the cross section in the axial direction of the light guide part 13. As shown in FIG.4 and FIG.5, the light guide part 13 has substantially a U-shape in the three dimensions when viewed from the top surface (the surface in the +z direction).

The inner surface of the light guide part 13 is covered with an infrared reflective film. The infrared reflective film in this embodiment is gold but is not limited to this. The infrared reflective film may be silver, aluminum, or a dielectric multilayer membrane. In addition, a protective film made of silicon oxide, silicon nitride, or the like may be formed on the infrared reflective film to prevent corrosion of the metal film of the infrared reflective film, if necessary. The infrared reflective film and the protective film can be formed by plating, sputtering, vacuum vapor deposition, or the like.

As shown by the bold arrows in FIG.5, the light guide part 13 reflects, on its inner infrared reflective film, infrared rays entering from the light source 2 and delivers the infrared rays to the light receiver 4, which has the light receiving surface on which the optical filter 3 is attached. Thus, the cover 1 serves as the optical path that efficiently guides infrared rays from the light source 2 to the light receiver 4 such that at least part of the reflected light reaches the light receiver 4 via the optical filter 3 by reflecting, on the infrared reflective film, the infrared rays emitted by the light source 2.

In this embodiment, the pipe-shaped light guide part 13 having a circular cross section serves as the light path in the cover 1. Such a light guide part 13 reflects infrared rays at a constant reflection angle in any direction in three dimensions (x axis, y axis, and z axis), regardless of the diameter of the cross section of the light guide part 13 or the path in the light guide part 13. Therefore, infrared rays emitted by the light source 2 can be reflected inside the light guide part 13 and efficiently enter the light receiver 4. For example, according to conventional reflective mirror-type optical gas sensor devices 100a and 100b, light having a specific angle can only be reflected to a desired position, and a loss of infrared rays occurs owing to misalignment of the light source and the light receiver.

The length of the optical path of infrared rays from the light source 2 to the light receiver 4 can be changed relatively easily by changing the diameter of the cross section of the light guide part 13.

The cover 1 is three-dimensionally designed and formed such that, on the board 6, a space S1 is formed below (in the -z direction) a portion of the light guide part 13 in the x-axis direction (FIG.2 and FIG.3). At least part of the signal processor 5 and the circuit elements 8 is placed in the space S1 and mounted on the board 6.

The cross-sectional area of the cross section of the light guide part 13 perpendicular to the axial direction is uniform in the axial direction. Since the cross-sectional area of the light guide part 13 is uniform, the gas concentration per unit volume of the detection target gas G entering in the light guide part 13 is easily equalized. Further, since infrared rays randomly passes instead of passing through a specific path, the optical gas sensor device 100 can easily react to changes in the gas concentration of the gas G. For example, the conventional reflective mirror-type optical gas sensor devices 100a, 100b, which have a fixed main optical path with respect to the inner volume of the cover, may not react to the gas unless the detection target gas enters the main optical path (unless the gas spreads over the entire cover).

As shown in FIG.3 and FIG.4, the cover part 110A has hollow sections 115, 116, 117A, 121, 122 as spaces for lightening. The cover part 110B has a hollow section 117B as a space for lightening. The hollow section 117B corresponds to the hollow section 117A. With the hollow sections 115, 116, 117A, 121, 122, and 117B, the cover 1 (optical gas sensor device 100) can be light-weighted.

As shown in FIG.4, the cover part 110B has fixing pins 123, 124. The fixing pins 123, 124 are protruding parts extending in the +z direction and fitted to not-illustrated recesses (internal holes) of the cover part 110A. By fitting the fixing pins 123, 124 into the recesses of the cover part 110A, the positions of the cover parts 110A, 110B are fixed, and the cover parts 110A, 100B are combined as one integral part.

As shown in FIG.2, the cover part 110A has gas intake ports (gas introduction holes) 111, 112 as gas introduction ports 11. The cover part 110B has gas intake ports (gas introduction holes) 113, 114 as gas introduction ports 11. The gas intake ports 111, 112 are holes formed in the -z direction from the top surface of the cover part 110A and penetrating to the light guide part 13. The gas intake port 113 is a hole formed in the +y direction from the -y direction side surface of the cover part 110B and penetrating to the space around the light source 2 (the space next to the light source 2). The space around the light source 2 communicates with the light guide part 13. The gas intake port 114 is a hole formed in the +y direction from the -y direction side surface of the cover part 110B and penetrating to the space around the optical filter 3 and the light receiver 4 (the space next to the optical filter 3 and the light receiver 4). The space around the optical filter 3 and the light receiver 4 communicates with the light guide part 13.

The gas intake ports 111, 112 directly communicate with the light guide part 13. Since part of the inner surface of the light guide part 13 is removed, the infrared-ray utilization efficiency is decreased. On the other hand, the gas intake port 113 indirectly communicates with the light guide part 13 through the space around the light source 2 and does not require damage on (removal of) part of the inner surface of the light guide part 13. Therefore, the infrared-ray utilization efficiency is increased. Similarly, the gas intake port 114 indirectly communicates with the light guide part 13 through the space around the optical filter 3 and the light receiver 4 and does not require damage on part of the inner surface of the light guide part 13. Therefore, the infrared-ray utilization efficiency is increased.

The shapes, sizes, and positions of the gas introduction ports 11 (gas intake ports 111, 112, 113, and 114) of the cover 1 in FIG.2 are examples and not limited to the examples. For example, the cover 1 may have only the gas intake ports 113, 114 and may not have the gas intake ports 111, 112. For another example, the gas intake port 113 may be formed on the -x side surface of the cover part 110B and the gas intake port 114 on the +x side surface of the cover 110B.

As shown in FIG.6, the light source 2 is a MEMS (Micro Electro Mechanical Systems) type light source mounted on the top surface (the +z side surface) of the board 6. The light source 2 has a membrane M having a membrane structure, for example. The light source 2 includes a silicon chip 21, a thin film heater 22, and a wire bonding pad 23. The silicon chip 21 is a semiconductor chip mainly made of silicon and has the membrane M in the center of the surface (x-y surface) of the silicon chip 21. The thin film heater 22 is a light source that emits infrared rays by being heated by energization. The thin film heater 22 is formed substantially at the center of the surface of the membrane M. The wire bonding pad 23 is wire-bonded to the wiring on the board 6.

The light source 2 as the MEMS type light source, which is small and low in height, contributes to a compact and especially low-in-height sensor module, as compared with a conventional incandescent light source or an LED (Light Emitting Diode).

Characteristically, the light source 2 as the MEMS type light source has a longer life, lower power consumption, and shorter response time as compared with a conventional light source. Reducing the power consumption of the light source, which is dominant to the current consumption of the entire sensor module, contributes to reducing the power consumption of the sensor module. The short response time of the MEMS light source contributes to shortening the standby time after energization when intermittent driving is performed, and thereby contributes to reducing average power consumption.

The light source 2 as the MEMS type light source can directly utilize the light emitted from the surface of a high-temperature part, as compared with a known light source. Such a light source 2 can be applied to the detection of gases having absorption bands at high wavelengths. The infrared ray emitting region of the light source 2 is patterned on the surface of the silicon substrate of the membrane M with high precision. Unlike a known incandescent light source constituted of a coiled filament, the light source 2 has very small individual variations in the emission direction. Therefore, when included in the sensor module, the light source 2 contributes to reducing variations in the light receiving amount and improving product yield. Since the light source 2 is manufactured in bulk from silicon wafers by the MEMS technology, the light source 2 is excellent in mass production.

The light source 2 is a surface-mounted component but is not limited to this. The light source 2 may be a DIP component (e.g., CAN package).

The optical filter 3 is mounted to cover the light receiving surface of the light receiver 4. The optical filter 3 transmits light (infrared rays) in a wavelength range (band) corresponding to the absorption wavelength specific to the detection target gas G. Thus, the transmission wavelength of the optical filter 3 is designed to match the absorption wavelength specific to the detection target gas G. Such an optical filter 3 suppresses changes in light quantities caused by gases other than the detection target gas G and improves the SN ratio of the detection signal of the light receiver 4. More specifically, the optical filter 3 filters infrared rays of a wide wavelength range, which are entering from the light source 2 after passing through the gas G (CO₂), and transmits infrared rays having a wavelength range corresponding to the absorption wavelength (4.26 µm) of the gas G.

The optical filter 3 includes, for example, a silicon substrate as a substrate and a dielectric multilayer film. The silicon substrate is a flat substrate made of silicon. The material of the substrate is not limited to silicon but can be Ge (germanium), quartz, alumina, BaF2 (barium fluoride), CaF2 (calcium fluoride), or the like. The dielectric multilayer film is made of layers of dielectrics and is provided on both sides of the silicon substrate. The planar shape of the optical filter 3 is rectangular but is not limited to this. The planar shape of the optical filter 3 may be other shapes, such as a circular shape.

The light receiver 4 is mounted on the +Z side surface of the board 6. The light receiver 4 is a thermopile-type light sensor (infrared sensor) having thermocouples. The light receiver 4 detects the amount of incident infrared rays and outputs a detection signal as an analog electric signal. However, the light receiver 4 is not limited to the thermopile-type infrared sensor but may be an infrared sensor of various types, as shown in the following TABLE I.

**[TABLE 1]**

| Theory of operation | | | Detection wavelength | Element material |
|---|---|---|---|---|
| Quantum type (Cooling type) | External photoelectric effect | Photoelectric tube | Ultra-violet rays | Silver cesium oxide (Ag-O-Cs) |
| | | | -0.9 *µ*m | Gallium Arsenide - Cesium(GaAs-Cs) |
| | Internal photoelectric effect | Photoconductive type | 3-5 µm | Mercury cadmium telluride(HgCdTe) |
| | | | | Indium antimonide(InSb) |
| | | | 8-12 µm | Mercury cadmium telluride(HgCdTe) |
| | | | | Gallium arsenide(GaAs) |
| | | | | Aluminum gallium arsenide(AlGaAs) |
| | | | | Quantum Well Infrared Photodetector(QWIP) |
| | | Photovoltaic type | 3-5 *µ*m | Platinum silicon(PrSi) |
| | | | | Indium antimonide(InSb) |
| | | | 8-12 *µ*m | Mercury cadmium telluride(HgCdTe) |
| | | | | Germanium silicon(GeSi) |
| Thermal type (Non-cooling type) | Pyroelectric effect | Pyroelectric element type | 1-3 *µ*m | Lead sulfide(PbS) |
| | | | 8-12*µ*m | Barium strontium titanate(BST) |
| | | | | Lead zirconate titanate(PZT) |
| | Thermoelectric effect | Thermocouple type | | Polycrystalline silicon(Poly-Si) |
| | Effect of change in electrical resistance by temperature | Bolometer type | | Vanadium oxide(VOx) |
| | | | | Giant Magneto Resistive Effect (CMR) |
| | | | | Yttrium-based superconductor(YBCO) |
| | | | | Amorphous silicon(a-Si) |

The light receiver 4 is a surface-mounted component but is not limited to this. The light receiver 4 may be a DIP component (e.g., CAN package). The light guide part 13 can efficiently obtain the light quantity regardless of the orientation of the light source 2 and light receiver 4, namely regardless of whether the light source 2 and light receiver 4 are surface-mounted components or DIP components.

The signal processor 5 is mounted on the +z side surface region of the board 6. The signal processor 5 is an AFE (Analog Front End) - IC (Integrated Circuit) as an electronic element (processor) that performs signal processing related to the detection signals of the light receiver 4. The signal processor 5 amplifies analog detection signals of the light receiver 4, performs AD conversion, and performs correction of individual variations in the optical gas sensor device 100 and so forth. By using the amplified digital detection signals, the signal processor 5 performs signal processing (calculation of a gas concentration or a value corresponding to the gas concentration and the state of the optical gas sensor device 100, generation of various signals thereof) and generates and outputs various digital signals.

The board 6 is a PCB (Printed Circuit Board) made of a glass epoxy resin plate or the like on which conductor wiring is printed. The cover 1, the light source **2,** the light receiver 4 on which the optical filter 3 is attached, the signal processor 5, the connector 7, and the circuit elements 8 are mounted on the top surface (+z side surface) of the board 6.

The connector 7 is mounted on the +z side surface region of the board 6 other than the cover 1 and the signal processor 5. The connector 7 outputs various digital signals, which are output by the signal processor 5, to an information processor of an apparatus (e.g., alarming apparatus) in the post stage. The connector 7 is connected to the information processor of the apparatus via a cable having a plug.

The circuit elements 8 are switches, chip resistors, chip capacitors, or the like mounted on the +z side surface region of the board 6.

Herein, a method of manufacturing the cover 1 is briefly described. First, the cover part 110A and the cover part 110B of the cover 1 are formed separately by resin injection molding with molds. The inner surface of the half-pipe section 118A of the cover part 110A and the inner surface of the half-pipe section 118B of the cover part 110B are smoothly formed by injection molding and do not need polishing.

Next, the infrared reflective film (and the protective film) is formed on the inner surfaces of the half-pipe sections 118A, 118B by plating (resin plating), sputtering, vacuum vapor deposition, or the like. Then, the fixing pins 123, 124 of the cover part 110B are fitted to the recesses of the cover part 110A to combine the cover parts 110A, 110B as one body. Thus, the cover 1 is manufactured.

As described above, according to the embodiment, the optical gas sensor device 100 includes: the light source 2 that emits infrared rays to the detection target gas G; the optical filter 3 that transmits infrared rays having a wavelength corresponding to an absorption wavelength of the detection target gas G; the light receiver 4 that detects the infrared ray entering via the optical filter 3 and generates a detection signal; and the cover 1 that covers the light source 2, the optical filter 3, and the light receiver 4. The cover 1 includes the light guide part 13 that guides infrared rays entering from the light source 2 to the light receiver 4 via the optical filter 3 by reflecting the infrared ray on the inner surface. The light guide part 13 has a pipe shape, and the cross section of the light guide part 13 perpendicular to the axial direction is circular.

Thus, the optical path can be freely formed in three dimensions, and detection accuracy for the detection target gas G can be increased. More specifically, since the light guide part 13 has a pipe shape with the circular cross section, the optical paths of infrared rays can be formed in any direction in three dimensions (x axis, y axis, and z axis), so that a long optical path is secured in a narrow space. Accordingly, detection accuracy of the detection target gas G can be increased. Further, since the direction of the optical path can be freely changed in three dimensions by the light guide part 13, space can be efficiently used, and the area of the board 6 can be reduced. Further, since the direction of the optical paths can be freely changed in three dimensions by the light guide part 13, the light quantity of infrared rays can be efficiently obtained regardless of whether the light source 2 and light receiver 4 are surface-mounted components or DIP components. Further, since the light guide part 13 has a pipe shape with the circular cross section, infrared rays are reflected at a constant angle on any part of the inner surface of the light guide part 13, so that all the infrared rays can be reflected. Thus, the optical gas sensor device 100 is resistant to misalignment of the light source 2 and the light receiver 4, and detection accuracy of the detection target gas G can be increased. Further, the length of the optical path can be changed relatively easily by changing the diameter of the circular cross section of the light guide part 13.

Further, the cover 1 includes the gas intake ports 111, 112, 113, and 114 that introduce the detection target gas G into the light guide part 13. The gas intake port 113 communicates with the space around the light source 2 and communicates with the light guide part 13 via the space. The gas intake port 114 communicates with the space around the light receiver 4 and communicates with the light guide part 13 via the space. With the gas intake ports 113, 114, the detection target gas G can be introduced to the light guide part 13 without damaging the inner surface of the light guide part 13. Thus, detection accuracy of the detection target gas G can be further increased.

The gas intake ports 111, 112 directly communicate with the light guide part 13. Thus, the detection target gas G can be directly introduced to the light guide part 13, and detection accuracy of the detection target gas G can be increased.

Further, the cross-sectional area of the cross section of the light guide part 13 is uniform in the axial direction. Therefore, the concentration of the detection target gas G can be equalized in the light guide part 13, and infrared rays randomly pass through the light guide part 13 instead of passing through a specific path in the light guide part 13. This allows the optical gas sensor device 100 to easily react to changes in the gas G (changes in the gas concentration of the gas G).

Further, the cover 1 includes the cover parts 110A, 110B into which the cover 1 is divided along a cross section of the light guide part 13 in the axial direction. The inner surface of the light guide part 13 of the cover parts 110A, 110B has an infrared reflective film. By forming the cover parts 110A,110B by resin injection molding, the inner surface of the light guide part 13 (the half-pipe sections 118A, 118B) does not need polishing. In this regard, the cover 1 (optical gas sensor device 100) can be easily manufactured with lower cost.

Further, the optical gas sensor device 100 includes the board 6 on which the light source 2, the light receiver 4, and the cover 1 are mounted. Part of the light guide part 13 (the part the axial direction of which is parallel to the x axis) is separate from the board 6. The cover 1 has the space S1 on the board 6 under the part of the light guide part 13, which is separate from the board 6. Thus, the signal processor 5 and the circuit elements 8 as mounted elements can be arranged in the space S1, and the light guide part 13 can be arranged above the signal processor 5 and the circuit elements 8. Thus, space can be efficiently used, and the area of the board 6 can be reduced.

The above embodiment is a preferred example of the optical gas sensor device according to the present invention and is not intended to limit the present invention.

For example, although the cover 1 is formed by resin injection molding in the above embodiment, the present invention is not limited to this. For example, the cover 1 may be made of metal as one body, such as aluminum, by a metal 3D printer. According to such a structure, the inner surface of the light guide part 13 of the cover 1 needs polishing, but the infrared reflective film (and the protective film) need not be formed. In this regard, the cover 1 (optical gas sensor device 100) can be easily formed. The inner surface of the light guide part 13 of the cover 1 is polished by electropolishing, polishing with beads, or the like.

Although the light guide part 13 of the cover 1 in the above embodiment has a pipe shape with a circular cross section perpendicular to the axial direction, the present invention is not limited to this. The light guide part 13 may have a pipe shape with an oval cross section perpendicular to the axial direction, for example. As with the light guide part 13 having a circular cross section, the light guide part 13 having an oval cross section can reduce limitation of reflection angles of optical paths of infrared rays and reduce the light quantity loss of infrared rays caused by misalignment of the light source 2 and the light receiver 4. Thus, the optical gas sensor device 100 is resistant to the misalignment, and detection accuracy of the detection target gas G can be increased.

Further, although the optical gas sensor device 100 in the above embodiment includes one set of the light source 2, the optical filter 3, the light receiver 4, and the light guide part 13, the present invention is not limited to this. The optical gas sensor device may include multiple sets of the light source 2, the optical filter 3, the light receiver 4, and the light guide part 13.

The detailed configuration and detailed operation of the optical gas sensor device 100 in the above embodiment may be appropriately modified without departing from the scope of the present invention.

### Industrial Applicability

As described above, the optical gas sensor device according to the present invention is suitable for detecting gases, such as CO₂.

### Reference Signs List

100, 100a, 100b Optical gas sensor device G Gas
1, 1a, 1b Cover
110A, 110B Cover parts
11 Gas introduction ports
111, 112, 113, 114 Gas intake ports
13 Light guide part
118A, 118B Half-pipe sections
115, 116, 117a, 121, 122, 117b Hollow sections
123, 124 Fixing pins
12 Gas filter
2, 2a, 2b Light source
21 Silicon chip
22 Thin film heater
23 Wire bonding pad
M Membrane
3 Optical filter
4, 4a, 4b Light receiver
5 Signal processor
6, 6a, 6b Board
7 Connector
8 Circuit elements

## Claims

1. An optical gas sensor device comprising:
a light source that emits infrared rays to a detection target gas;
an optical filter that transmits infrared rays having a wavelength corresponding to an absorption wavelength of the detection target gas;
a light receiver that detects infrared rays entering via the optical filter and generates a detection signal; and
a cover that covers the light source, the optical filter, and the light receiver, wherein
the cover includes a light guide part that guides infrared rays entering from the light source to the light receiver via the optical filter by reflecting the infrared rays on an inner surface;
the light guide part has a pipe shape; and
a cross section of the light guide part perpendicular to an axial direction is circular or oval.

2. The optical gas sensor device according to claim 1, wherein the cover includes a gas introduction part that introduces the detection target gas into the light guide part.

3. The optical gas sensor device according to claim 2, wherein the gas introduction part communicates with at least either a space around the light source or a space around the light receiver and communicates with the light guide part via the space.

4. The optical gas sensor device according to claim 2, wherein the gas introduction part directly communicates with the light guide part.

5. The optical gas sensor device according to claim 1, wherein a cross-sectional area of the cross section of the light guide part is uniform in the axial direction.

6. The optical gas sensor device according to claim 1, wherein
the cover has multiple cover parts into which the cover is divided along a cross section of the light guide part in the axial direction; and
an inner surface of the light guide part of the multiple cover parts has an infrared reflective film.

7. The optical gas sensor device according to claim 1, wherein the cover is made of metal and formed as one body.

8. The optical gas sensor device according to any one of claims 1 to 7, further comprising a board on which the light source, the light receiver, and the cover are mounted, wherein
at least part of the light guide part is separate from the board; and
the cover has a space on the board under the part of the light guide part, which is separate from the board.
